# EUROPÄISCHE PATENTSCHRIFT

(11) **EP 3 763 723 B1**
(45) Veröffentlichungstag und Bekanntmachung des Hinweises auf die Patenterteilung: **13.04.2022**
(21) Anmeldenummer: 19185649.1
(22) Anmeldetag: 11.07.2019
(51) Int. Cl.: C07F 9/6574, C07C 45/50, C07C 47/02, B01J 31/12

(54) **BISPHOSPHITE MIT EINEM GESCHLOSSENEN UND EINEM OFFENEN FLÜGELBAUSTEIN**
BIS-PHOSPHITES WITH A CLOSED AND AN OPEN BLADE MODULE
BISPHOSPHITES COMPORTANT UN COMPOSANT CENTRALE FERMÉ ET D'UN COMPOSANT CENTRALE OUVERTE

(43) Veröffentlichungstag der Anmeldung: 13.01.2021
(73) Patentinhaber: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); QUELL, Thomas, 2030 Antwerpen (BE)
(74) Vertreter: Evonik Patent Association

(56) Entgegenhaltungen:
- ANNEMIEK VAN ROOY ET AL: "Bulky Diphosphite-Modified Rhodium Catalysts: Hydroformylation and Characterization", ORGANOMETALLICS, Bd. 15, Nr. 2, 1. Januar 1996 (1996-01-01) , Seiten 835-847, XP55655992, US ISSN: 0276-7333, DOI: 10.1021/om950549k

## Beschreibung

Die Erfindung betrifft Bisphosphite, die einen geschlossenen und einen offenen Flügelbaustein aufweisen. Einer der beiden Flügelbausteine weist also eine C-C-Brücke zwischen den beiden Phenylresten auf, der andere der beiden Flügelbausteine nicht.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In A. van Rooy et al., "Bulky Diphosphite-Modified Rhodium Catalysts: Hydroformylation and Characterization", Organometallics, 1996, 15(2), Seiten 835-847, werden unterschiedliche Liganden zur Hydroformylierung beschrieben. Hierunter auch der folgende Ligand:

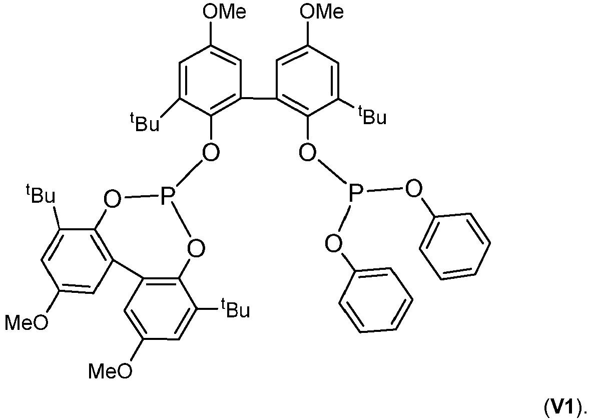

Die technische Aufgabe der Erfindung ist die Bereitstellung neuer Liganden, welche in der Hydroformylierung von Olefinen eine gesteigerte Ausbeute verglichen zu dem aus dem Stand der Technik bekannten Liganden aufweisen.

Die Aufgabe wird gelöst durch eine Verbindung gemäß Anspruch 1.

Verbindung der Formel (I): 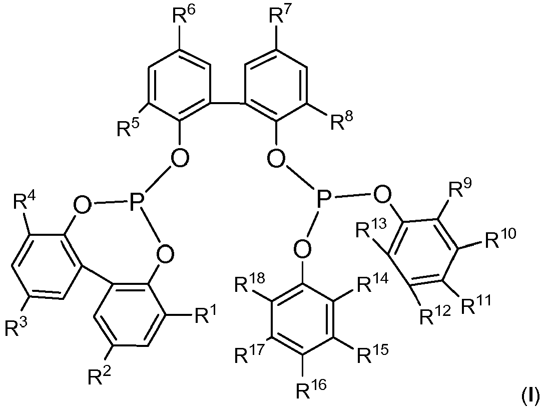 wobei
R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸ ausgewählt sind aus: -H, -(C₁-C₁₂)-Alkyl, -O-(C₁-C₁₂)-Alkyl, Halogen, -NH2, -NO₂;
wobei mindestens einer der Reste R¹⁰, R¹², R¹⁵, R¹⁷ nicht für -H steht.

In einer Ausführungsform stehen mindestens zwei der Reste R¹⁰, R¹², R¹⁵, R¹⁷ nicht für -H.

In einer Ausführungsform stehen mindestens zwei der Reste R¹⁰, R¹², R¹⁵, R¹⁷ für -(C₁-C₁₂)-Alkyl.

In einer Ausführungsform stehen zwei der Reste R¹⁰, R¹², R¹⁵, R¹⁷ für -(C₁-C₁₂)-Alkyl und die anderen beiden für -H.

In einer Ausführungsform stehen zwei der Reste R¹⁰, R¹², R¹⁵, R¹⁷ für -CH₃ und die anderen beiden für -H.

In einer Ausführungsform stehen R² und R³ für -O-(C₁-C₁₂)-Alkyl.

In einer Ausführungsform stehen R² und R³ für -O-CH₃.

In einer Ausführungsform stehen R⁶ und R⁷ für -O-(C₁-C₁₂)-Alkyl.

In einer Ausführungsform stehen R⁶ und R⁷ für -O-CH₃.

In einer Ausführungsform stehen R¹ und R⁴ für -(C₁-C₁₂)-Alkyl.

In einer Ausführungsform stehen R¹ und R⁴ für -^{t}Bu.

In einer Ausführungsform stehen R⁵ und R⁸ für -(C₁-C₁₂)-Alkyl.

In einer Ausführungsform stehen R⁵ und R⁸ für -^{t}Bu.

In einer Ausführungsform weist die Verbindung die Struktur (1) oder (2) auf:

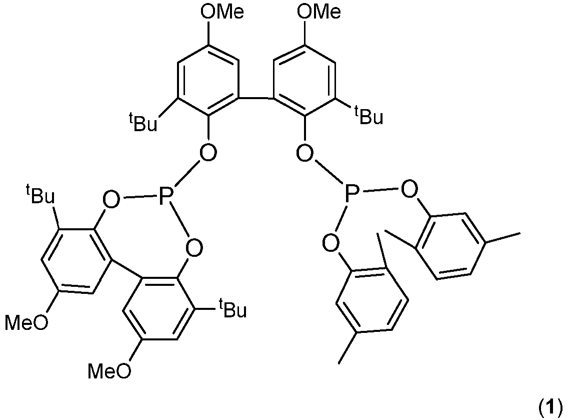

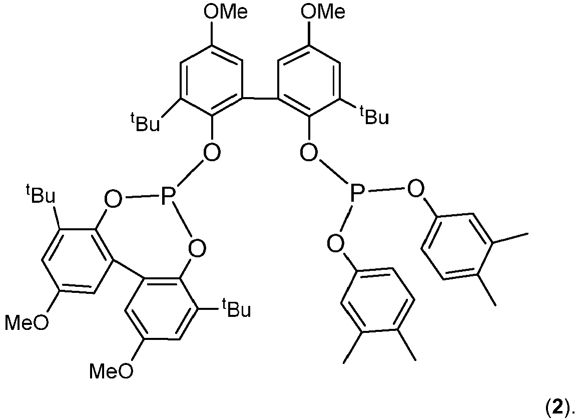

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 70 bar.

Besonders bevorzugt sind eine Temperatur von 100 °C bis 140 °C und ein Druck von 40 bis 60 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Otefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden n-Olefine endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Allgemeine Synthese

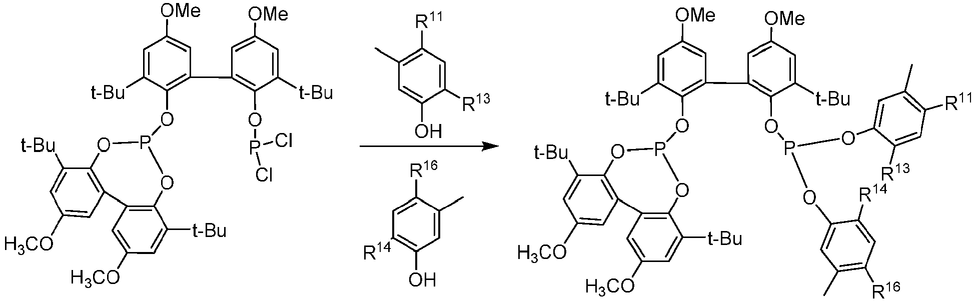

In einem sekurierten Schlenk wurde das entsprechend substituierte Phenol abgewogen und über Nacht mittels Ölpumpenvakuum bei Raumtemperatur nachgetrocknet. Am nächsten Morgen wurde unter Rühren Toluol und entgastes Triethylamin bei Raumtemperatur zugegeben. Zu dieser Phenol-Triethylamin-Lösung wurde dann langsam und stetig bei Raumtemperatur das Dichlorophosphit hinzugegeben. Die Temperatur wurde hierbei langsam auf 60 °C angehoben. Anschließend wurde das Reaktionsgemisch auf 70 °C gebracht und 2 Tage lang gerührt. Das erhaltene Produkt wurde gewaschen und gefrittet.

Reinheit: 92 bis 96%, Ausbeute: 70 bis 92%.

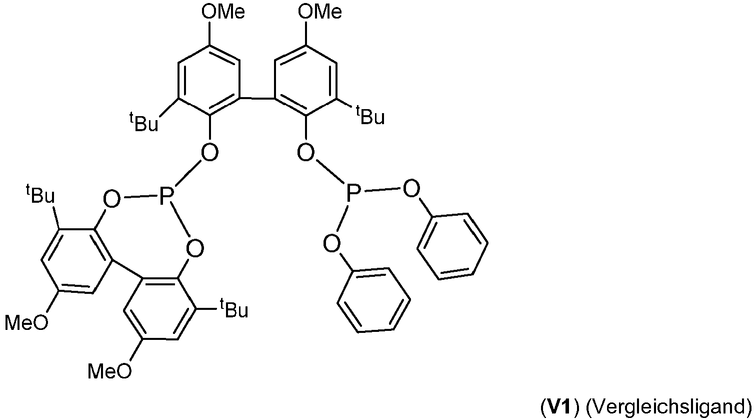

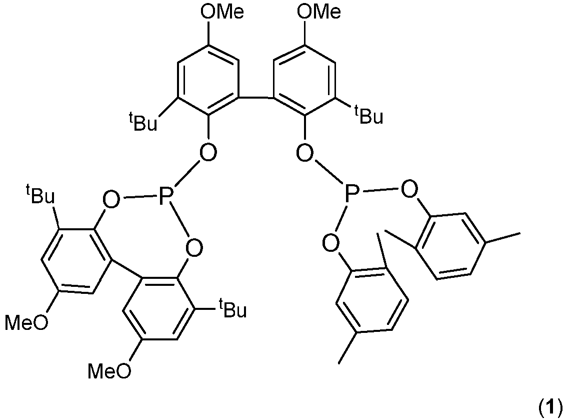

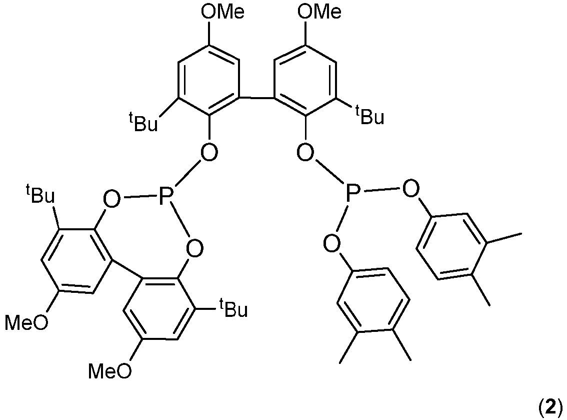

### Katalvseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzte Gemisch der n-Octene (Oxeno GmbH, Octenisomerengemisch aus 1-Octen: 3 %; cis+trans-2-Octen: 49 %; cis+trans-3-Octen: 29 %; cis+trans-Octen-4: 16 %; gerüstisomere Octene: 3 %) wurden über Natrium am Rückfluß erhitzt und unter Argon destilliert.

Im Autoklaven wurden unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Von der Rhodiumverbindung wurden 10 ml einer 4,31 millimolaren Lösung in den Autoklaven gegeben. Anschließend wurde die entsprechende Menge an Liganden (Rh/Ligand = 1:5) in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurden die n-Octene (10,70 g; 95,35 mmol) eingefüllt. Der Autoklav wurde bei einem Gesamtgasdruck (Synthesegas: Linde; H₂ (99,999%) : CO (99,997%) = 1:1) von 42 bar aufgeheizt. Nach Erreichen der Reaktionstemperatur von 120 °C wurde der Synthesegasdruck auf 48,5 bar erhöht und das Olefin mit einem in der Druckpipette eingestellten Überdruck von ca. 3 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

### Ergebnisse der Katalvseversuche

**Tabelle 1: Hydroformylierung der n-Octene**

| Eintrag | Ligand | Ausbeute [%] |
|---|---|---|
| 1 | **V1** | 15 |
| 2 | **1*** | 17 |
| 3 | **2*** | 19 |

| | | |
|---|---|---|
| * erfindungsgemäße Verbindung | | |

Mit den erfindungsgemäßen Verbindungen **(1)** und **(2)** konnte jeweils eine gegenüber dem Vergleichsliganden **(V1)** gesteigerte Ausbeute erzielt werden.

Die durchgeführten Versuche belegen, dass die gestellte Aufgabe durch die erfindungsgemäßen Verbindungen gelöst wird.

## Patentansprüche

1. Verbindung der Formel (I): 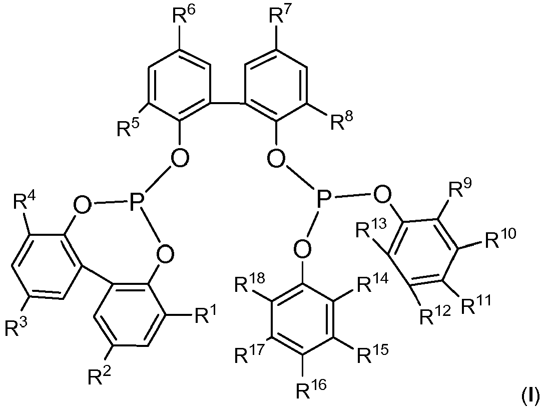 wobei
R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸ ausgewählt sind aus: -H, -(C₁-C₁₂)-Alkyl, -O-(C₁-C₁₂)-Alkyl, Halogen, -NH2, -NO₂;
wobei mindestens einer der Reste R¹⁰, R¹², R¹⁵, R¹⁷ nicht für -H steht.

2. Verbindung nach Anspruch 1,
wobei mindestens zwei der Reste R¹⁰, R¹², R¹⁵, R¹⁷ nicht für -H steht.

3. Verbindung nach einem der Ansprüche 1 bis 2,
wobei mindestens zwei der Reste R¹⁰, R¹², R¹⁵, R¹⁷ für -(C₁-C₁₂)-Alkyl stehen.

4. Verbindung nach einem der Ansprüche 1 bis 3,
wobei zwei der Reste R¹⁰, R¹², R¹⁵, R¹⁷ für -(C₁-C₁₂)-Alkyl stehen und die anderen beiden für -H.

5. Verbindung nach einem der Ansprüche 1 bis 4,
wobei zwei der Reste R¹⁰, R¹², R¹⁵, R¹⁷ für -CH₃ stehen und die anderen beiden für -H.

6. Verbindung nach einem der Ansprüche 1 bis 5,
wobei R² und R³ für -O-(C₁-C₁₂)-Alkyl stehen.

7. Verbindung nach einem der Ansprüche 1 bis 6,
wobei R⁶ und R⁷ für -O-(C₁-C₁₂)-Alkyl stehen.

8. Verbindung nach einem der Ansprüche 1 bis 7,
wobei R¹ und R⁴ für -(C₁-C₁₂)-Alkyl stehen.

9. Verbindung nach einem der Ansprüche 1 bis 8,
wobei R⁵ und R⁸ für -(C₁-C₁₂)-Alkyl stehen.

10. Verbindung nach einem der Ansprüche 1 bis 9,
wobei die Verbindung die Struktur (**1**) oder (**2**) aufweist:

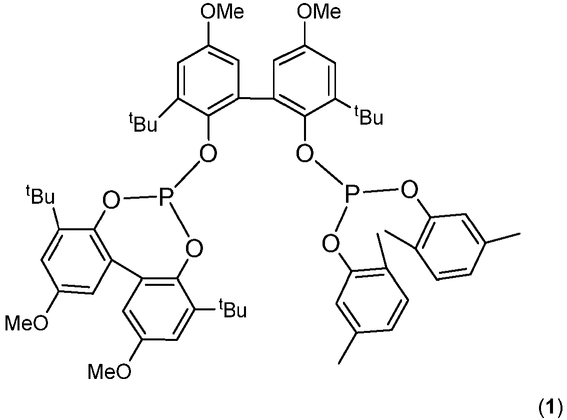

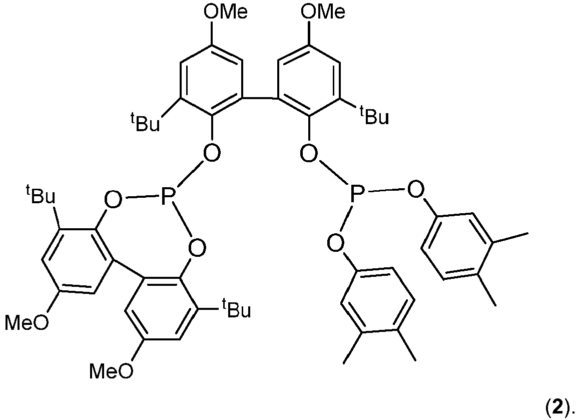

11. Verwendung einer Verbindung nach Anspruch 1 bis 10,
in einem Ligand-Metall-Komplex zur Katalyse einer Hydroformylierungsreaktion.

12. Verfahren umfassend die Verfahrensschritte:
a) Vorlegen eines Olefins,
b) Zugabe einer Verbindung nach einem der Ansprüche 1 bis 10 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound of the formula (I): 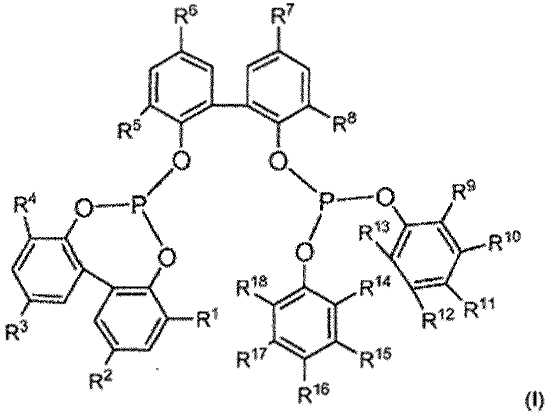 wherein
R¹, R², R³, R⁴, R⁵, R⁶, R⁷, R⁸, R⁹ R¹⁰, R¹¹, R¹², R¹³, R¹⁴, R¹⁵, R¹⁶, R¹⁷, R¹⁸ are selected from: -H, -(C₁-C₁₂)-alkyl, -O- (C₁-C₁₂) -alkyl, halogen, -NH₂, -NO₂;
wherein at least one of the radicals R¹⁰, R¹², R¹⁵, R¹⁷ is other than -H.

2. Compound according to Claim 1,
wherein at least two of the radicals R¹⁰, R¹², R¹⁵, R¹⁷ are other than -H.

3. Compound according to either of Claims 1 and 2,
wherein at least two of the radicals R¹⁰, R¹², R¹⁵, R¹⁷ are -(C₁-C₁₂)-alkyl.

4. Compound according to any of Claims 1 to 3,
wherein two of the radicals R¹⁰, R¹², R¹⁵, R¹⁷ are - (C₁-C₁₂) -alkyl and the other two are -H.

5. Compound according to any of Claims 1 to 4,
wherein two of the radicals R¹⁰, R¹², R¹⁵, R¹⁷ are -CH₃ and the other two are -H.

6. Compound according to any of Claims 1 to 5,
wherein R² and R³ are -O-(C₁-C₁₂)-alkyl.

7. Compound according to any of Claims 1 to 6,
wherein R⁶ and R⁷ are -O-(C₁-C₁₂)-alkyl.

8. Compound according to any of Claims 1 to 7,
wherein R¹ and R⁴ are - (C₁-C₁₂) -alkyl.

9. Compound according to any of Claims 1 to 8,
wherein R⁵ and R⁸ are - (C₁-C₁₂) -alkyl.

10. Compound according to any of Claims 1 to 9, wherein the compound has the structure (1) or (2):

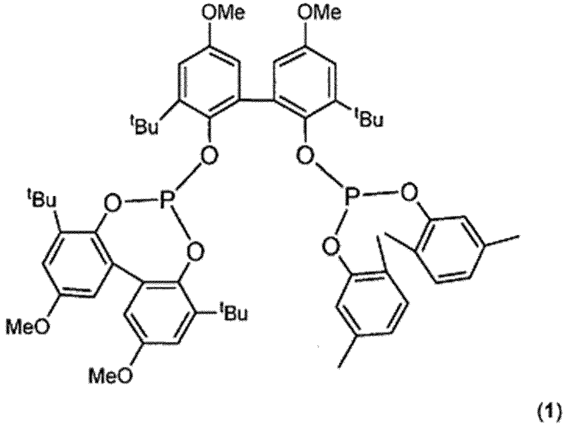

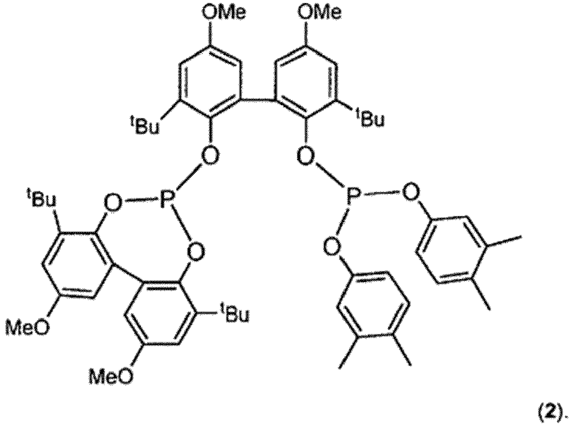

11. Use of a compound according to Claims 1 to 10 in a ligand-metal complex for the catalysis of a hydroformylation reaction.

12. Process comprising the process steps of:
a) initially charging an olefin,
b) adding a compound according to any of Claims 1 to 10 and a substance comprising a metal selected from: Rh, Ru, Co, Ir,
c) feeding in H₂ and CO,
d) heating the reaction mixture of a) to c), wherein the olefin is converted to an aldehyde.

## Revendications

1. Composé de formule (I) : 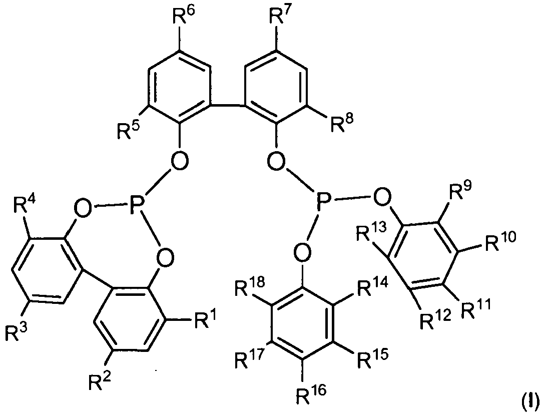 dans laquelle
R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸ sont choisis parmi : -H, - (C₁-C₁₂) -alkyle, -O-(C₁-C₁₂)-alkyle, halogène, -NH₂, -NO₂ ;
au moins un des radicaux R¹⁰, R¹², R¹⁵, R¹⁷ ne représentant pas -H.

2. Composé selon la revendication 1,
au moins deux des radicaux R¹⁰, R¹², R¹⁵, R¹⁷ ne représentant pas -H.

3. Composé selon l'une quelconque des revendications 1 à 2,
au moins deux des radicaux R¹⁰, R¹², R¹⁵, R¹⁷ représentant - (C₁-C₁₂) -alkyle.

4. Composé selon l'une quelconque des revendications 1 à 3,
au moins deux des radicaux R¹⁰, R¹², R¹⁵, R¹⁷ représentant -(C₁-C₁₂)-alkyle et les deux autres représentant -H

5. Composé selon l'une quelconque des revendications 1 à 4,
au moins deux des radicaux R¹⁰, R¹², R¹⁵, R¹⁷ représentant -CH₃ et les deux autres représentant -H

6. Composé selon l'une quelconque des revendications 1 à 5,
R² et R³ représentant -O-(C₁-C₁₂)-alkyle.

7. Composé selon l'une quelconque des revendications 1 à 6,
R⁶ et R⁷ représentant -O- (C₁-C₁₂) -alkyle.

8. Composé selon l'une quelconque des revendications 1 à 7,
R¹ et R⁴ représentant - (C₁-C₁₂) -alkyle.

9. Composé selon l'une quelconque des revendications 1 à 8,
R⁵ et R⁸ représentant - (C₁-C₁₂) -alkyle.

10. Composé selon l'une quelconque des revendications 1 à 9,
le composé présentant la structure (1) ou la structure (2) :

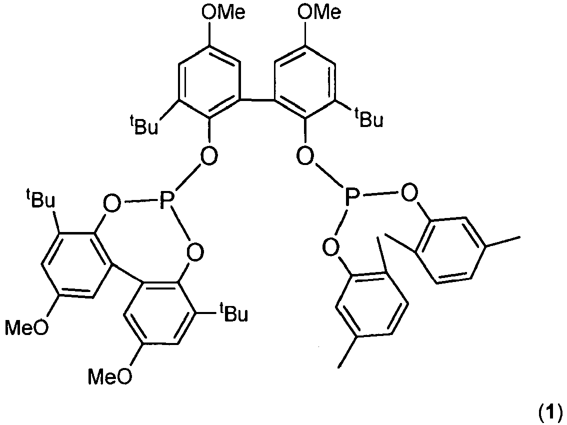

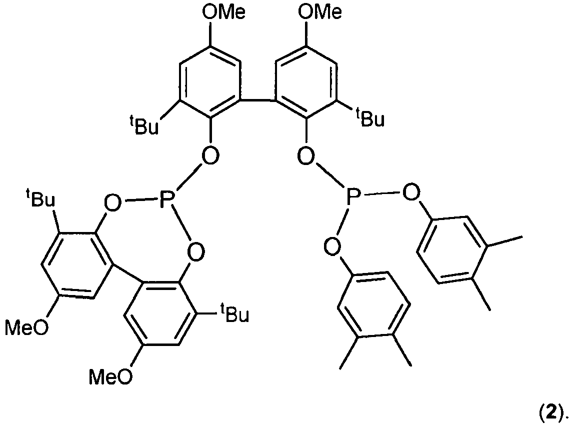

11. Utilisation d'un composé selon la revendication 1 à 10 dans un complexe ligand-métal pour la catalyse d'une réaction d'hydroformylation.

12. Procédé comprenant les étapes de procédé :
a) disposition préalable d'une oléfine,
b) ajout d'un composé selon l'une quelconque des revendications 1 à 10 et d'une substance qui présente un métal choisi parmi : Rh, Ru, Co, Ir,
c) introduction de H₂ et de CO,
d) chauffage du mélange réactionnel de a) à c), l'oléfine étant transformée en aldéhyde.
